# EUROPEAN PATENT SPECIFICATION

(11) **EP 2 877 147 B1**
(45) Date of publication and mention of the grant of the patent: **31.10.2018**
(21) Application number: 13741742.4
(22) Date of filing: 24.07.2013
(51) Int. Cl.: A61K 8/34, A61K 8/41, A61K 8/42, A61Q 5/12, A61K 8/04

(54) **Process for making a conditioning gel phase**
Prozess zur Herstellung einer konditionierenden Gelphase
Procédé de préparation d'une phase gélifiée conditionnante

(30) Priority: 27.07.2012 EP 12178165
(43) Date of publication of application: 03.06.2015
(73) Proprietor: Unilever PLC, London, Greater London EC4Y 0DY (GB); Unilever N.V., 3013 AL Rotterdam (NL)
(72) Inventor: CASUGBO, Christia, Bebington Wirral Merseyside CH63 3JW (GB); FLANAGAN, Mark, Bebington Wirral Merseyside CH63 3JW (GB); HOUGH, John, Alan, Bebington Wirral Merseyside CH63 3JW (GB); NAUGHTON, John, Michael, Bebington Wirral Merseyside CH63 3JW (GB); SERRIDGE, David, Wirral Merseyside CH62 2FD (GB)
(74) Representative: James, Helen Sarah
(86) International application number: PCT/EP2013/065645
(87) International publication number: WO 2014/016351

(56) References cited:
- EP-A1- 1 491 180
- WO-A1-99/62492
- WO-A1-2010/136285
- US-A- 4 726 945
- US-A1- 2006 078 527

## Description

The present invention relates to a process for manufacturing improved conditioner compositions.

WO 99/62492 A1 discloses a method of making a hair conditioning composition and is considered as prior art closest to the claimed subject-matter.

WO 2010/136285 (Unilever) discloses a process whereby molten fatty alcohol and mineral oil is mixed into an aqueous composition comprising lactic acid, stearamidopropyl dimethyl amine and BTAC.

US 2006/078527 (Sanjeev) and US 4 726 945 (Patel) disclose a multi phase personal care composition comprising stearamidopropyl dimethylamine glutamate and adding fatty alcohol.

Despite the prior art there remains a need for improved conditioning compositions.

Accordingly, and in a first aspect, there is provided a process for making a conditioning gel phase according to claim 1.

A conditioning composition made using a conditioning gel phase of the invention has been shown to be superior to compositions made by standard processes where the materials are mixed in water at around 70°C. The superior conditioning manifests itself in superior conditioner thickness (despite having lower solids levels) and next day clean feel and conditioning benefits. These are surprising since it would be expected that superior conditioning products usually leave the hair lank and greasy the following day sue to excessive deposition of solids.

The temperature of the mixture of the aqueous isotropic solution and fatty alcohol is maintained at from 55°C to 65°C.

The molten fatty alcohol is added to the aqueous isotropic solution of cationic surfactant.

In this process the temperature of the mixture is controlled by modifying the temperature/rate of the mixture of the fatty alcohol and the cationic surfactant solution. The temperature needs to be carefully controlled in order to achieve the right conditioning gel phase structure. The improvement thus resides in the balance of thermal energy at the point of mixing the fatty alcohol with the isotropic mixture.

After formation of the gel phase further water and additional ingredients may be added in one go or it may be staged. Preferably the gel phase is cooled prior to addition of the water.

The conditioning composition ultimately made using such conditioning gel phase has improved conditioning capabilities.

Preferably, the temperature of the mixture of the fatty alcohol and aqueous isotropic solution is maintained at from 58°C to 62°C; most preferably at 60°C.

Preferably, and prior to addition to the isotropic mixture, the fatty alcohol is maintained at a temperature sufficient to maintain the fatty alcohol in a liquid phase. Preferably the fatty alcohol is maintained at from 80°C to 85°C.

Preferably, the resulting conditioning gel phase is mixed with a mixer having a rotor tip speed of 10-34, preferably from 21-27 and especially preferably 24 ms-1.

Preferably, the fatty alcohol comprises from 8 to 22 carbon atoms, more preferably 16 to 22. Fatty alcohols are typically compounds containing straight chain alkyl groups. Examples of preferred fatty alcohols include cetyl alcohol, stearyl alcohol and mixtures thereof.

The level of fatty alcohol in the conditioner of the invention (not just the conditioning gel phase) will generally range from 0.01 to 10%, preferably from 0.1 % to 8%, more preferably from 0.2 % to 7 %, most preferably from 0.3 % to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Suitable conditioning components include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g. oleyl). Alkyl groups are optionally ethoxylated on the alkyl chain with one or more ethyleneoxy groups.

Suitable cationic surfactants for use in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Preferably the conditioning composition formed from the conditioning gel phase of the invention comprises from 0.1 wt% to 10 wt% of the total composition of quaternary ammonium surfactant of the formula N⁺R¹R²R³R⁴ as described above, more preferably from 0.2 wt% to 5 wt%.

Preferably the aqueous isotropic solution comprises from 1 to 5 wt% of quaternary ammonium surfactant.

Preferably, the aqueous isotropic solution comprises from 1 to 5% wt. amidoamine surfactant.

Suitable cationic amidoamine surfactants are preferably of the general formula (I):

(I) R1CONH(CH2)mN(R2)R3

in which R¹ is a hydrocarbyl chain having 10 or more carbon atoms, R² and R³ are independently selected from hydrocarbyl chains of from 1 to 10 carbon atoms, and m is an integer from 1 to about 10; and

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

The isotropic solution also comprises neutralizer for any amidoamine present. Preferred neutralizers include acid. Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.
Particularly preferred is lactic acid.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

Should an amidoamine of the type described herein be present then the corresponding acid component is present in the aqueous isotropic solution.

Preferably composition comprises from 0.1 wt% to 10 wt% of the total composition of protonated amidoamine as described above, more preferably from 0.2 wt% to 5 wt%.

In a preferred embodiment the weight ratio of protonated amidoamine to quaternium ammonium surfactant is from 1:2 to 2:1.

In conditioning compositions of the invention (not merely the conditioning gel phase), the total level of cationic surfactant will generally range from 0.01 % to 10%, more preferably 0.05 % to 7.5%, most preferably 0.1 % to 5% by weight of the composition.

Preferably, the molten fatty alcohol is added to the aqueous cationic surfactant. More preferably, it is added gradually to the cationic surfactant.

In a second aspect there is provided a process for manufacturing a conditioning composition by forming a conditioning gel phase obtained by the first aspect and then adding any remaining ingredients. Typical remaining ingredients include fragrances, silicones, fibre actives or other benefit agents.

Preferably, the conditioning composition is mixed with a mixer with rotor tip speed of 10-34, preferably from 21-27 and especially preferably 24 ms-1 one more time after the remaining ingredients have been added.

Suitable mixers for use with the invention have a kw/kg figures preferably in the range from 2 to 30 kw/kg, more preferably 10 - 25 and even more preferably 15-25.

Conditioning compositions of the invention or using conditioning gel phases of the invention also deposit silicone better than conventionally made conditioning compositions.

Accordingly, the compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25°C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of 0.15 micron are generally termed microemulsions.

Emulsified silicones for use in the conditioner compositions of the invention will typically have a size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone".

Specific examples of amino functional silicones suitable for use in the invention are the aminosilicone oils DC2-8220, DC2-8177 and DC2-8566 (all ex Dow Corning).

Suitable quaternary silicone polymers are described in EP-A-0 530 974. A preferred quaternary silicone polymer is K3474 ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

### EXAMPLE

| **Material** | **Active Level** | **A** | **1** |
|---|---|---|---|
| Stearylamidopropyl dimethylamine | 100 | 1.25 | 1 |
| Behentrimonium Chloride | 70 | 1.25 | 1 |
| Lactic Acid | 88 | 0.38 | 0.285 |
| Cetearyl Alcohol | 100 | 5 | 4 |
| Parfum | 100 | 0.6 | 0.6 |
| Preservative | 55 | 0.1 | 0.1 |
| Disodium EDTA | 100 | 0.1 | 0.1 |
| Preservative | 1.5 | 0.04 | 0.04 |
| Potassium Chloride | 100 | 0.1 | 0.1 |
| Dimethicone/amodimethicone/Cetrimonium Chloride | 70 | 3.57 | 3.57 |
| Water | | To 100 | To 100 |

Formulation A is made by standard process which involves mixing the BTAC and fatty alcohol in water at around 70C. In contrast, formulation 1 is made by adding cationic surfactants to water at 60°C, maintain temperature by use of external heating, and stir.

Gradually add molten (85°C) fatty alcohol to this mixture, maintain temperature at 60°C by use of external heating or cooling, and stir.

Cool this towards ambient by adding more water, and other ambient temperature ingredients, and use of external cooling if required, and stir.

The compositions have different levels of conditioning active to demonstrate the improved conditioning performance of the composition made by the claimed process.

| **Panel data** | A | 1 |
|---|---|---|
| **Conditioner Attribute** | | |
| Conditioner thickness | 63.19 B | 74.61 A |
| Ease of styling | 70.79 bc | 75.08 a |
| Level Conditioning | 64.37 C | 70.48 AB |
| Overall Styling | 67.38 BC | 72.64 A |

| **Next Day** | | |
|---|---|---|
| ND clean feel | 61.96 D | 69.74 AB |
| ND conditioning | 59.33 C | 67.23 A |

Panel data with approx 40 panellists with dry damaged hair. Assessment via line scale. (5 different products tested in total vs. control).

The data shows that using a better process we have a thicker product despite having lower total solids (i.e. FA and BTAC). The ingredients are being used more efficiently.

In addition, the product is both significantly more conditioning than the control as well as feeling significantly more clean next day- unusual because there is usually a trade off (more conditioning= heavier) again, despite having a lower level of solids, i.e. conditioning active. One would have expected that a composition which provided improved conditioning benefits immediately post application would achieve this through increased deposition. However, if this were the case, the next day benefits would be markedly reduced.

## Claims

1. Process for making a conditioning gel phase comprising:
i) forming an aqueous isotropic solution of cationic surfactant, wherein the isotropic solution also comprises neutralizer when amidoamine is present;
ii) adding to the aqueous isotropic solution of cationic surfactant molten fatty alcohol
wherein the temperature during mixing the fatty alcohol with the isotropic cationic surfactant solution is maintained from 55°C to 65°C and wherein the fatty alcohol has a straight chain alkyl group comprising from 8 to 22 carbons and wherein the cationic surfactant comprises a cationic surfactant of the formula N+R1R2R3R4 wherein R1, R2, R3 and R4 are independently C1 to C30 alkyl or benzyl,
iii) and thereafter adding any remaining ingredients.

2. Process according to any preceding claim in which the isotropic cationic surfactant solution comprises from 1 to 5 wt% of protonated amidoamine surfactant.

3. Process according to any preceding claim in which the composition comprises from 0.2 to 7 wt% of the total composition of fatty alcohol.

4. Process according to any preceding claim in which the cationic surfactant comprises behenyltrimethylammonium chloride.

5. Process according to any preceding claim in which the fatty alcohol is is a C16 to C22 alcohol.

6. Process according to any preceding claim wherein the resulting mixture is mixed with a mixer with rotor tip speed of 10-34 ms-1.

7. Process for manufacturing a conditioning composition by adding any remaining ingredients to a conditioning gel phase obtained by any of claims 1 to 6.

8. Process according to claim 7 comprising mixing the composition with a mixer with rotor tip speed of 10-30 ms-1.

## Patentansprüche

1. Verfahren zur Herstellung einer konditionierenden Gelphase, umfassend:
i) Ausbilden einer wässrigen isotropen Lösung kationischen Tensids, wobei die isotrope Lösung auch ein Neutralisationsmittel umfasst, wenn Amidoamin vorliegt,
ii) Zugeben eines geschmolzenen Fettalkohols zu der wässrigen isotropen Lösung kationischen Tensids,
wobei die Temperatur während des Mischens des Fettalkohols mit der isotropen Lösung kationischen Tensids von 55°C bis 65°C aufrechterhalten wird und wobei der Fettalkohol eine geradkettige Alkyl-Gruppe aufweist, die von 8 bis 22 Kohlenstoffatome umfasst, und wobei das kationische Tensid ein kationisches Tensid der Formel N+R1R2R3R4 umfasst, wobei R1, R2, R3 und R4 unabhängig C1- bis C30-Alkyl oder Benzyl darstellen,
iii) und danach Zugeben aller übrigen Bestandteile.

2. Verfahren nach irgendeinem vorhergehenden Anspruch, in welchem die isotrope Lösung kationischen Tensids von 1 bis 5 Gew.-% protoniertes Amidoamin-Tensid umfasst.

3. Verfahren nach irgendeinem vorhergehenden Anspruch, in welchem die Zusammensetzung von 0,2 bis 7 Gew.-% der gesamten Zusammensetzung an Fettalkohol umfasst.

4. Verfahren nach irgendeinem vorhergehenden Anspruch, in welchem das kationische Tensid Behenyltrimethylammoniumchlorid umfasst.

5. Verfahren nach irgendeinem vorhergehenden Anspruch, in welchem der Fettalkohol ein C16- bis C22-Alkohol ist.

6. Verfahren nach irgendeinem vorhergehenden Anspruch, wobei die erhaltene Mischung mit einem Mischer mit einer Rotorspitzengeschwindigkeit von 10-34 ms⁻¹ gemischt wird.

7. Verfahren zur Herstellung einer konditionierenden Zusammensetzung unter Zugeben aller übrigen Bestandteile zu einer konditionierenden Gelphase, erhalten nach irgendeinem der Ansprüche 1 bis 6.

8. Verfahren nach Anspruch 7, umfassend das Mischen der Zusammensetzung mit einem Mischer mit einer Rotorspitzengeschwindigkeit von 10-30 ms⁻¹.

## Revendications

1. Procédé de fabrication d'une phase de gel de conditionnement comprenant :
i) la formation d'une solution isotrope aqueuse de tensioactif cationique, dans lequel la solution isotrope comprend également un neutralisant lorsqu'une amidoamine est présente ;
ii) l'addition à la solution isotrope aqueuse d'alcool gras fondu de tensioactif cationique
dans lequel la température pendant le mélange de l'alcool gras avec la solution de tensioactif cationique isotrope est maintenue à de 55°C à 65°C et dans lequel l'alcool gras présente un groupe alkyle linéaire comprenant de 8 à 22 atomes de carbone et dans lequel le tensioactif cationique comprend un tensioactif cationique de la formule N+R1R2R3R4 dans lequel R1, R2, R3 et R4 sont indépendamment un groupe alkyle ou benzyle en C1 à C30,
iii) et après cela l'addition des ingrédients restants.

2. Procédé selon l'une quelconque des revendications précédentes dans lequel la solution de tensioactif cationique isotrope comprend de 1 à 5 % en masse de tensioactif d'amidoamine protonée.

3. Procédé selon l'une quelconque des revendications précédentes dans lequel la composition comprend de 0,2 à 7 % en masse de la composition totale d'alcool gras.

4. Procédé selon l'une quelconque des revendications précédentes dans lequel le tensioactif cationique comprend du chlorure de béhényltriméthylammonium.

5. Procédé selon l'une quelconque des revendications précédentes dans lequel l'alcool gras est un alcool en C16 à C22.

6. Procédé selon l'une quelconque des revendications précédentes dans lequel le mélange résultant est mélangé avec un mélangeur avec une vitesse de tige de rotor de 10-34 ms-1.

7. Procédé de fabrication d'une composition de conditionnement par addition des ingrédients restants à une phase de gel de conditionnement obtenue par l'une quelconque des revendications 1 à 6.

8. Procédé selon la revendication 7 comprenant le mélange de la composition avec un mélangeur avec une vitesse de tige de rotor de 10-30 ms-1.
